# EUROPÄISCHE PATENTSCHRIFT

(11) **EP 1 625 215 B1**
(45) Veröffentlichungstag und Bekanntmachung des Hinweises auf die Patenterteilung: **13.12.2006**
(21) Anmeldenummer: 04739133.9
(22) Anmeldetag: 05.05.2004
(51) Int. Cl.: C12N 9/02, C12Q 1/26

(54) **VERFAHREN ZUR HERSTELLUNG EINES HYDROXYLIERUNGSKATALYSATORS UND SEINE VERWENDUNG**
METHOD FOR PRODUCING A HYDROXYLATION CATALYST AND THE USE THEREOF
PROCEDE DE PRODUCTION D'UN CATALYSEUR D'HYDROXYLATION ET SON UTILISATION

(30) Priorität: 09.05.2003 DE 10321082
(43) Veröffentlichungstag der Anmeldung: 15.02.2006
(73) Patentinhaber: BASF Aktiengesellschaft, 67056 Ludwigshafen (DE)
(72) Erfinder: HAUER, Bernhard, 67136 Fussgönheim (DE); HABICHER, Tilo, 67346 Speyer (DE); SCHMID, Rolf, 70329 Stuttgart (DE); MAURER, Steffen, Christian, 73525 Schwäbisch Gmünd (DE); URLACHER, Vlada, Beniaminovna, 70374 Stuttgart (DE); SCHULZE, Holger, 73614 Schorndorf (DE); HUBER, Norbert, 79539 Lörrach (DE); BACHMANN, Till, T., 70569 Stuttgart (DE)
(86) Internationale Anmeldenummer: PCT/EP2004/004748
(87) Internationale Veröffentlichungsnummer: WO 2004/099398

(56) Entgegenhaltungen:
- IWUOHA, EMMANUEL I. ET AL: "Reactivities of organic phase biosensors 3: electrochemical study of cytochrome P450cam immobilized in a methyltriethoxysilane sol-gel" ELECTROANALYSIS , 12(12), 980-986 CODEN: ELANEU; ISSN: 1040-0397, 2000, XP009034944
- GILL IQBAL ECKERT HELLMUT (AV.-PROP.) ET AL: CHEMISTRY OF MATERIALS, , 13(10), 3404-3421, 261 REFS. ISSN: 0897-4756, 2001, XP002291608
- MAURER S C ET AL.: "Immobilisation of P450 BM-3 and an NADP+ Cofactor Recycling System: Towards a Technical Application of Heme-Containing Monooxygenases in Fine Chemical Synthesis" ADVANCED SYNTHESIS AND CATALYSIS, Bd. 345, Nr. 6-7, Juni 2003 (2003-06), Seiten 802-810, XP002291609
- URLACHER V B ET AL: "Microbial P450 enzymes in biotechnology" APPLIED MICROBIOLOGY AND BIOTECHNOLOGY, Bd. 64, Nr. 3, April 2004 (2004-04), Seiten 317-325, XP002291610 ISSN: 0175-7598
- MILES^A C S ET AL: "Protein engineering of cytochromes P-450" BIOCHIMICA ET BIOPHYSICA ACTA. PROTEIN STRUCTURE AND MOLECULAR ENZYMOLOGY, ELSEVIER, AMSTERDAM,, NL, Bd. 1543, Nr. 2, 29. Dezember 2000 (2000-12-29), Seiten 383-407, XP004279114 ISSN: 0167-4838

## Beschreibung

Die vorliegende Erfindung betrifft ein Verfahren zur Herstellung eines Hydroxylierungskatalysators auf Basis einer Cytochrom P450 Monooxygenase, sowie Verfahren zur Hydroxilierung organischer Substrate mittels dieser Hydroxylierungkatalysatoren.

Cytochrom P450 Monooxygenasen (im folgenden CYP genannt) katalysieren die Hydroxylierung einer Reihe hydrophober Substrate an aktivierten und nicht-aktivierten Kohlenstoffatomen. Dabei wird ein Sauerstoffatom von O₂ in das Substrat eingebaut, während das andere Sauerstoffatom zu H₂O reduziert wird unter gleichzeitiger Oxidation von Nicotin-Adenin-Dinukleotid (Phosphat) (NAD(P)H). Diese Hydroxilierung geschieht in vielen Fällen regio- und stereospezifisch.

Eine besonders vielversprechende Cytochrom P450 Monooxygenase ist das ursprünglich aus Bacillus megaterium klonierte Gen, im folgenden CYP BM-3 genannt. Es handelt sich dabei um ein 119 kDa großes natürliches Fusionsprotein, das aus einer Hämenthaltenden Monooxygenase-Domäne und einer FAD- und FMN-enthaltenden Reduktase-Domäne zusammengesetzt ist (L.P.Wen, A.J. Fulco, J. Biol. Chem.1987, 262, 6676-6682; A.J.Fulco, R.T.Ruettinger, Life Sci. 1987, 40, 1769-1775).

Die natürlichen Substrate für CYP BM-3 sind langkettige Fettsäuren (C12-C20), die ausschliesslich an den subterminalen Positionen ω-1, ω-2, ω-3, teilweise mit hoher Enantioselektivität hydroxiliert werden. Varianten des CYP BM-3 (sogenannte Muteine) zeigen auch eine Aktivität gegenüber nicht-natürlichen Substraten wie kurzkettige Fettsäuren (Ost et al. FEBS Lett. 2000, 486, 173-177 ; Li et al. Biochim. Biophys. Acta 2001, 1545, 114-121), Indolen (Li et al. Chemistry 2000, 6, 1531-1536), polyzyklischen aromatischen Kohlenwasserstoffen (Li et al. Appl. Environ. Microbiol. 2001, 67, 5735-5739), Alkanen (Appel et al. J. Biotechnol. 2001, 88, 167-171) und Styrolen (Li et al. FEBS Lett. 2001, 508, 249-252).

IWUOHA, EMMANUEL I. ET AL: "Reactivities of organic phase biosensors 3: electrochemical study of cytochrome P450cam immobilized a methyltriethoxysilane sol-gel" ELECTROANALYSIS, 12(12), 980-986 beschreibt eine Cytochrom P450 Monooxygenase (Cytrochrom P450_{cam}), welche an einem Methyltriethoxysilan Sol-Gel-System immobilisiert ist und als Biosensor für z.B. Campher verwendet werden kann.

Einer Anwendung dieser CYP im größeren technischen Maßstab stehen immer noch die Probleme der geringen Stabilität und der Produktabtrennung entgegen. Ein weiterer Nachteil ist die Abhängigkeit der CYP von teuren Cofaktoren wie NADPH.

Diese Probleme lassen sich teilweise dadurch lösen, daß die CYP immobilisiert für die Hydroxilierungsreaktionen eingesetzt werden. Allerdings wird durch viele Immobilisierungsverfahren das Enzym zumindest teilweise inaktiviert oder es besteht diffusionskontrolliert eine limitierte Versorgung des Enzyms mit benötigten Cofaktoren und Substrat.

Es bestand daher die Aufgabe, ein Verfahren bereitzustellen, daß es erlaubt CYP mit hoher enzymatischer Aktivität zu immobilisieren.
Gegenstand der Erfindung ist ein Verfahren zur Herstellung eines Hydroxylierungskatalysators indem man
i) eine Cytochrom P450 Monooxygenase in einer Sol-Gel Matrix einbettet,
ii) ein enzymatisches NADPH-Regenerationssystem in einer Sol-Gel Matrix einbettet,
und beide Komponenten i) und ii) zusammenfügt, sofern sie nicht schon vor der Einbettung zusammengemischt waren.

Cytochrom P450 Monooxygenasen (CYP) und ihr Einsatz bei Biotransformationen sind dem Fachmann beispielsweise aus E.T. Farinas et al. Adv. Synth. Catal. 2001, 343, 601-606 oder V. Urlacher and R.D. Schmid Curr. Opin. Biotechnol. 2002, 13, 557-564 bekannt.

Insbesondere sind solche CYP für das erfindungsgemäße Verfahren gut geeignet, die ursprünglich aus Mikroorganismen, insbesondere solchen der Gattung Bacillus isoliert worden sind.

Eine besonders gut geeignete Cytochrom P450 Monooxygenase ist das ursprünglich aus Bacillus megaterium klonierte Protein, im folgenden CYP BM-3 genannt. Es handelt sich dabei um ein 119 kDa großes natürliches Fusionsprotein, das aus einer Hämenthaltenden Monooxygenase-Domäne und einer FAD- und FMN-enthaltenden Reduktase-Domäne zusammengesetzt ist (L.P.Wen, A.J. Fulco, J. Biol. Chem.1987, 262, 6676-6682; A.J.Fulco, R.T.Ruettinger, Life Sci. 1987, 40, 1769-1775).

Ausgehend von diesem CYP-BM-3 können Muteine durch rekombinante DNA Techniken produziert werden, die an bestimmten Aminosäurepositionen Veränderungen gegenüber dem wildtyp CYP BM-3 tragen.

Besonders für das erfindungsgemäße Verfahren geeignete Muteine sind solche, die an der Position 74 eine andere Aminosäure als Ala tragen, und/oder an der Position 87 eine andere Aminosäure als Phe tragen und/oder an der Position 188 eine andere Aminosäure als Leu tragen und/oder an der Position 386 eine andere Aminosäure als Phe tragen. Die Veränderungen an den aufgeführten Positionen können entweder als Einzelmutationen oder auch kumuliert als Mehrfachmutationen eingeführt werden.

Ein solches, wegen seiner großen Substratbreite besonders gut geeignetes Mutein ist dasjenige,das gegenüber der wildtyp CYP BM-3die folgenden drei Aminosäuresubstitutionen besitzt: Position 74 Ala ersetzt durch Gly, Position 87 Phe ersetzt durch Val, Pos 188 Leu ersetzt durch Gln. Dieses Mutein kann insbesondere für die Hydroxilierung von Alkanen und Aromaten eingesetzt werden.

Ein weiteres besonders für die Hydroxilierung von β-Ionon geeignetes Mutein ist dasjenige,das gegenüber der wildtyp CYP BM-3die folgenden drei Aminosäuresubstitutionen besitzt: Position 74 Ala ersetzt durch Glu, Position 87 Phe ersetzt durch Val, Pos 386 Phe ersetzt durch Ser.

Die Einbettung von Enzymen in Sol-Gel Matrices ist in einem Übersichtsartikel von I.Gill, Chem. Mater. 2001, 13, 3404-3421 beschrieben.

Besonders geeignete Sol-Gel Matrices für das erfindungsgemäße Verfahren sind solche auf Kieselsäure Basis. Besonders geeignete Sol-Gel Matrices können aus Alkoxysilanen, insbesondere aus Tetraalkoxysilanen vor allem Tetraethoxysilane (TEOS) und Tetramethoxysilane (TMOS) hergestellt werden. Zur Herstellung der Sol-Gel-Matrices und der Einbettung von CYP wird auf den o.g. Artikel von Gill verwiesen (I.Gill, Chem. Mater. 2001, 13, 3404-3421) auf den hiermit in vollem Umfang Bezug genommen wird.

Das enzymatische NADPH-Regenerationssystem (im folgenden NADPH -RS genannt) besteht aus einem NAD⁺ bzw NADP⁺ abhängigen Enzym, das ein Substrat zu einem Produkt oxidiert unter gleichzeitiger Reduktion des NAD⁺ zu NADH bzw. des NADP⁺ zu NADPH. Geeignet sind dafür prinzipiell alle NAD⁺ bzw. NADP⁺ abhängigen Dehydrogenasen, insbesondere mikrobielle Dehydrogenasen und insbesondere Formiatdehydrogenasen.

Eine bevorzugte Ausführungsform des NADPH-RS ist die NADP⁺ abhängige Formiatdehydrogenase (EC 1.2.1.2) im folgenden mit FDH abgekürzt aus Pseudomonas sp. 101. FDH katalysyiert die NAD⁺ abhängige Oxidation von Formiat zu CO₂. Die Vorteile dieses Systems liegen bei den geringen Substratkosten (Formiat) und der leichten Entfernbarkeit des entstehenden Produkts (CO₂). Eine mutierte Form der FDH weist eine hohe Aktivität mit NADP⁺ auf und kann daher besonders gut als NADPH regenerierendes Enzym in Kombination mit CYP eingesetzt werden. Diese besonders gut geeignete mutierte Form der FDH ist von Tishkov et al. Biotechnol. Bioeng. 1999, 64, 187-193 sowie von Seelbach et al. Tetrahedron Lett. 1996, 37, 1377-1380 beschrieben. In diesen genannten Dokumenten ist sowohl die natürliche als auch die mutierte FDH beschrieben, sowie Verfahren zu ihrer Herstellung, insbesonders ihrer rekombinanten Expression in E. coli.

Die Einbettung von CYP und NADPH-RS in eine Sol-Gel Matrix kann gleichzeitig oder in getrennten Ansätzen erfolgen. Unter gleichzeitiger Einbettung versteht man, daß zuerst eine Zubereitung von CYP, bevorzugt eine Lösung von CYP, mit einer Zubereitung von NADPH-RS, bevorzugt einer Lösung von NADPH-RS, vereinigt wird und diese vereinigte Zubereitung anschließend in die Sol-Gel Matrix eingebettet wird.

Unter einer getrennten Einbettung ist zu verstehen, daß eine Zubereitung von CYP, bevorzugt eine Lösung von CYP, in eine Sol-Gel Matrix eingebettet wird und in einem zweiten Ansatz eine Zubereitung von NADPH-RS, bevorzugt einer Lösung von NADPH-RS, in eine Sol-Gel Matrix eingebettet wird und anschließend diese beiden Ansätze vereinigt werden.

Bevorzugt verwendet wird für das erfindungsgemäße Verfahren die getrennte Einbettung, da bei ihr auch das stöchiometrische Verhältnis von CYP zu NADPH-RS noch im Nachhinein flexibel einstellbar ist.

Ein weiterer Gegenstand der Erfindung sind Zubereitungen, die eine CYP und ein NADPH-RS eingebettet in einer Sol-Gel Matrix enthalten. Solche Zubereitungen sind nach den oben beschriebenen Verfahren herstellbar. Diese Zubereitungen haben den Vorteil, daß es damit gelingt, Hydroxylierungskatalysatoren in stabiler, gut lagerfähiger Form herzustellen und für den Einsatz bei spezifischen Hydroxilierungsreaktionen bereitzustellen.

Ein weiterer Gegenstand der Erfindung sind Verfahren zur enzymatischen Hydroxylierung eines Substrates unter Verwendung eines der erfindungsgemäß herstellbaren Hydroxylierungskatalysatoren. Als Substrat geeignet sind eine Vielzahl von organischen Verbindungsklassen, insbesondere langkettige Fettsäuren (C12-C20), die insbesonders an den subterminalen Positionen ω-1, ω-2, ω-3 hydroxiliert werden, aber auch kurzkettige Fettsäuren (Ost et al. FEBS Lett. 2000, 486, 173-177 ; Li et al. Biochim. Biophys. Acta 2001, 1545, 114 - 121), Indole (Li et al. Chemistry 2000, 6, 1531-1536), polyzyklische aromatische Kohlenwasserstoffe (Li et al. Appl. Environ. Microbiol. 2001, 67, 5735-5739), Alkane (Appel et al. J. Biotechnol. 2001, 88, 167-171) und Styrole (Li et al. FEBS Lett. 2001, 508, 249-252).

Die Umsetzung dieser Substrate mit dem erfindungsgemäß hergestellten Hydroxylierungskatalysator erfolgt unter dem Fachmann für enzymatische Reaktionen bekannten Bedingungen. Die Reaktion kann in einem breiten Temperaturbereich zwischen 0 und 70, bevorzugt zwischen 5 und 50 und besonders bevorzugt zwischen 10 und 40°C durchgeführt werden.

Das zu hydroxylierende Substrat kann in einem organischen oder wäßrigen Lösungsmittel gelöst oder suspendiert vorliegen, bei flüssigen Substraten kann unter Umständen auch ganz auf die Zugabe von Lösungsmitteln verzichtet werden. Bevorzugt für die Umsetzung ist ein Gemisch aus wäßrigen und organischen Lösungsmitteln, insbesonders DMSO/Wasser. Besonders geeignet sind solche DMSO/Wasser Gemische bei denen DMSO 1-10% v/v beträgt.

Die Hydroxilierungsreaktion kann batchweise oder kontinuierlich durchgeführt werde. In einer bevorzugten Ausführungsform mit FDH als NADPH-RS wird die Reaktion kontinuierlich durchgeführt, wobei neben dem zu hydroxilierenden Substrat kontinuierlich auch Formiationen der Reaktion zugeführt werden und das hydroxylierte Produkt sowie das entstehende CO₂ kontinuierlich der Reaktion entnommen werden.

Die Erfindung wird durch die folgenden Beispiele weiter veranschaulicht.

### Aktivität und Stabilität der Sol-Gel immobilisierten CYP BM-3

Als Modellreaktion wurde die Hydroxilierung von p-Nitrophenoxydecansäure (10-pNCA) benutzt, die einen leichten photometrischen Nachweis des gebildeten Produkts p-Nitrophenolat erlaubt (Schwaneberg et al. Anal. Biochem. 1999, 269, 359-366).

Nach Zugabe zur Reaktionsmischung bildet das immobilisierte Enzym eine trübe Mischung. Daher waren direkte kinetische Versuche zur Aktivitätsbestimmung des Sol-Gel-eingebetteten CYP BM-3 nicht möglich. Daher wurde die Aktiviät des Sol-Gel-eingebetteten CYP BM-3 durch Inkubation aller Komponenten des Standard pNCA Tests für eine bestimmte Zeitspanne bestimmt und anschliessendes Zentrifugieren um das gelbe Reaktionsprodukt vom festen Katalysator abzutrennen, bestimmt. Aus dem Überstand wurde dann die Absorption bei 410 nm bestimmt.

Untersuchungen zur Langzeitstabilität ergaben, daß die im Sol-Gel eingebettete CYP BM-3 bei 4°C während 36 Tage keinen Aktivitätsverlust erlitt. Das freie Enzym wies eine Halbwertszeit von 26 Tagen auf bei Lagerung in 50 mM KPi und eine Halbwertszeit von 288 Tagen bei Stabilisierung mit 50% Glycerol. Die Halbwertszeit des Sol-Gel eingebetteten Enzyms ist beträchlich länger als diese 288 Tage.

Das erfindungsgemäß immobilisierte Enzym weist auch bei 25°C eine bemerkenswert hohe Stabilität auf. Bei dieser Temperatur wird die Halbwertszeit auf 29 Tage bestimmt.

Eine weitere selektive Hydroxilierungsreaktion wurde mit dem Sol-Gel-eingebetteten CYP BM-3 am Substrat n-Octan durchgeführt. Es wurden 79% des Eduktes hydroxiliert. Erhalten wurden die Regioisomeren 2-Oktanol, 3-Oktanol und 4-Oktanol in einem molaren Verhaltnis von 1:2,1:1,6 (Gaschromatografisch nachgewiesen).

Eine weitere selektive Hydroxilierungsreaktion wurde mit dem Sol-Gel-eingebetteten CYP BM-3 am Substrat Naphtalin durchgeführt. Es wurden 77% des Eduktes hydroxiliert. Als Hauptprodukt wurde 1-Naphtol (85%) und als Nebenprodukt 2-Naphtol (15%) erhalten (Gaschromatografisch nachgewiesen).

### Cofaktor Regeneration

Zwei Möglichkeiten der erfindungsgemäßen Cofaktor-Regenerierung wurden untersucht. In einer ersten Reihe von Experimenten wurde die Co-immobilisierung von beiden Enzymen (CYP und FDH) untersucht, in einer zweiten Reihe von Experimenten wurden beide Enzyme separat immobilisiert und anschliessend im Verhältnis 1:1 (m/m) zusammengemischt. Die Einbettung erfolgte in beiden Fällen in eine TEOS Sol-Gel Matrix.

Co-immobilisierte Enzyme aus der ersten experimentellen Reihe wurden in die p-NCA-Test gegeben mit einem zehnfachen Überschuß von p-NCA über oxidiertes NADP⁺. Die Aktivität der separat immobilisierten FDH in Kombination mit separat immobilisierte CYP war beträchtlich höher als bei den co-immobilisierten Enzymen.

Nach drei Stunden Reaktionszeit zeigten die co-immobilisierten Enzyme einen Umsatz von bis zu 28% der pNCA Umsetzung, während die Mischung aus separat immobilisierten Enzymen zu einer Umsetzung von bis zu 75% führte.

Diese Ergebnisse machen es möglich, einen kontinuierlich arbeitenden Bioreaktor auf Basis eines Sol-Gel eingebetteten CYP mit NADPH-RS zu betreiben.

## Patentansprüche

1. Verfahren zur Herstellung eines Hydroxylierungskatalysators indem man
i) eine Cytochrom P450 Monooxygenase in einer Sol-Gel Matrix einbettet,
ii) ein enzymatisches NADPH-Regenerationssystem in einer Sol-Gel Matrix einbettet,
und beide Komponenten i) und ii) zusammenfügt, sofern sie nicht schon vor der Einbettung zusammengemischt waren.

2. Verfahren nach Anspruch 1, **dadurch gekennzeichnet, daß** als Monooxygenasen Enzyme , die ursprünglich aus der Gattung Bacillus isoliert worden sind, verwendet.

3. Verfahren nach Anspruch 2, **dadurch gekennzeichnet, daß** die Monooxygenasen aus Bacillus megaterium isoliert worden sind.

4. Zubereitungen enthaltend eine Cytochrom P450 Monooxygenase und ein enzymatisches NADPH-Regenerationssystem in einer Sol-Gel Matrix.

5. Verfahren zur enzymatischen Hydroxylierung eines Substrates, indem man das Substrat mit einem Hydroxylierungskatalysator, herstellbar gemäß einem der Ansprüche 1 bis 3 umsetzt.

6. Verfahren nach Anspruch 5, **dadurch gekennzeichnet, daß** als Substrat β-Ionon eingesetzt wird.

## Claims

1. A process for preparing a hydroxylation catalyst by
i) embedding a cytochrome P450 monooxygenase in a sol-gel matrix,
ii) embedding an enzymatic NADPH-regenerating system in a sol-gel matrix,
and combining the two components i) and ii) unless they were already mixed together before the embedding.

2. The process according to claim 1, wherein enzymes which had originally been isolated from the genus Bacillus are used as monooxygenases.

3. The process according to claim 2, wherein the monooxygenases have been isolated from Bacillus megaterium.

4. A preparation comprising a cytochrome P450 monooxygenase and an enzymatic NADPH-regenerating system in a sol-gel matrix.

5. A process for the enzymatic hydroxylation of a substrate by reacting the substrate with a hydroxylation catalyst which can be prepared according to any of claims 1 to 3.

6. The process according to claim 5, wherein β-ionone is employed as substrate.

## Revendications

1. Procédé de préparation d'un catalyseur d'hydroxylation, dans lequel
i) on enrobe une cytochrome P450 monooxygénase dans une matrice de sol-gel,
ii) on enrobe un système de régénération enzymatique de NADPH dans une matrice de sol-gel,
et on assemble les deux composants i) et ii) dans la mesure où ils n'ont pas déjà été mélangés ensemble avant l'enrobage.

2. Procédé suivant la revendication 1, **caractérisé en ce que**, comme monooxygénases, on utilise des enzymes qui ont été originellement isolées à partir du genre Bacillus.

3. Procédé suivant la revendication 2, **caractérisé en ce que** les monooxygénases ont été isolées à partir de Bacillus megaterium.

4. Compositions contenant une cytochrome P450 monooxygénase et un système de régénération enzymatique de NADPH dans une matrice de sol-gel.

5. Procédé d'hydroxylation enzymatique d'un substrat, dans lequel on fait réagir le substrat avec un catalyseur d'hydroxylation qui peut être préparé selon l'une des revendications 1 à 3.

6. Procédé suivant la revendication 5, **caractérisé en ce que**, comme substrat, on met en oeuvre de la β-ionone.
